# EUROPEAN PATENT APPLICATION

(11) **EP 4 129 389 A1**
(43) Date of publication of application: **08.02.2023**
(21) Application number: 21780833.6
(22) Date of filing: 31.03.2021
(51) Int. Cl.: A61M 25/14, A61B 5/01

(54) **CATHETER**

(30) Priority: 31.03.2020 JP 2020064895
(71) Applicant: Toray Industries, Inc., Tokyo 103-8666 (JP)
(72) Inventor: SAKAKIBARA, Hajime, Otsu-shi, Shiga 520-2141 (JP); OKADA, Tatsuya, Otsu-shi, Shiga 520-2141 (JP); MATSUKUMA, Akinori, Otsu-shi, Shiga 520-2141 (JP); MATSUMOTO, Hiroshi, Otsu-shi, Shiga 520-2141 (JP); NAKAJIMA, Hiroki, Otsu-shi, Shiga 520-2141 (JP)
(74) Representative: Kador & Partner PartG mbB
(86) International application number: PCT/JP2021/013779
(87) International publication number: WO 2021/201077

(57) **Abstract**

A catheter includes a first tubular portion provided with a first opening communicating with an interior of the first tubular portion; a second tubular portion passing through the first tubular portion, the second tubular portion having an extension extending from a leading end of the first tubular portion and provided with a second opening communicating with an interior of the second tubular portion; and a temperature sensor configured to measure temperature of a measuring unit placed in the extension of the second tubular portion. The catheter collects fluid discharged from one of the first opening and the second opening from another one of the first opening and the second opening.

## Description

### TECHNICAL FIELD

The present invention relates to a catheter.

### BACKGROUND ART

In catheter ablation known as a treatment for arrhythmia such as atrial fibrillation, an ablation catheter is inserted into the body, and a leading end of the ablation catheter is heated or cooled to ablate a site of interest in the heart. In this catheter ablation, there is a possibility that heat of ablation is transmitted to an internal organ near the heart such as the esophagus and damages the internal organ.

Patent Literature 1 discloses an invention of a temperature measurement catheter which is inserted into an internal organ such as the esophagus when catheter ablation is performed. According to the invention disclosed in Patent Literature 1, internal temperature of the esophagus or the like is monitored, and when the internal temperature is high or low, the catheter ablation is temporarily stopped to provide a cooling time.

### CITATION LIST

### PATENT LITERATURE

Patent Literature 1: JP 2016-119936 A

### SUMMARY OF THE INVENTION

### OBJECT OF THE INVENTION

However, the aforementioned approach takes time and potentially causes an increase in patient burden. For this reason, it is required to efficiently control temperature of an internal organ.

The present invention has been made in light of such problems, and an object of the invention is to provide a catheter capable of controlling temperature of an internal organ efficiently.

### SOLUTION TO PROBLEM

A catheter according to the invention includes: a first tubular portion provided with a first opening communicating with an interior of the first tubular portion; a second tubular portion passing through the first tubular portion, the second tubular portion having an extension extending from a leading end of the first tubular portion and provided with a second opening communicating with an interior of the second tubular portion; and a temperature sensor configured to measure temperature of a measuring unit placed in the extension of the second tubular portion, in which the catheter is configured to collect fluid discharged from one of the first opening and the second opening from another one of the first opening and the second opening.

In the catheter, the measuring unit may be placed between the first opening and the second opening in a longitudinal direction of the catheter.

In the catheter, the catheter may be configured to collect the fluid discharged from the second opening from the first opening.

In the catheter, the first opening may open at the leading end of the first tubular portion.

In the catheter, the second opening may include a plurality of openings disposed on a side surface of the extension of the second tubular portion, the openings being separated from each other along a central axis of the second tubular portion.

In the catheter, the temperature sensor may have a plurality of the measuring units placed alternately with the second opening along the central axis of the second tubular portion.

In the catheter, one of the plurality of openings of the second opening may have an area larger than an area of another opening disposed on the proximal side of the one opening.

In the catheter, the second opening may include a plurality of openings disposed on a side surface of the extension of the second tubular portion, the openings being separated from each other in a circumferential direction around the central axis of the second tubular portion.

In the catheter, the first tubular portion and the second tubular portion may be integrated.

In the catheter, the extension of the second tubular portion may have a corrugated shape curved toward one side and another side.

In the catheter, the second tubular portion may be movable relative to the first tubular portion and configured to be housed in the first tubular portion.

In the catheter, the corrugated shape may has a first curved portion curved toward the one side, a second curved portion curved toward the other side and an intermediate portion between the first curved portion and the second curved portion, and the measuring unit may be placed in the first curved portion, the second curved portion and the intermediate portion, respectively.

Furthermore, a catheter according to the invention includes: a first tubular portion provided with a first opening communicating with an interior of the first tubular portion; a second tubular portion having an extension extending beyond a leading end of the first tubular portion and provided with a second opening communicating with an interior of the second tubular portion; and a temperature sensor configured to measure temperature of a measuring unit placed in the extension of the second tubular portion, in which the catheter is configured to collect fluid discharged from one of the first opening and the second opening from another one of the first opening and the second opening.

### ADVANTAGEOUS EFFECTS OF THE INVENTION

According to the invention, it is possible to efficiently control temperature of an internal organ.

### BRIEF DESCRIPTION OF DRAWINGS

FIG. 1 is a schematic view of a catheter according to a first embodiment.
FIG. 2 is an enlarged cross-sectional view of a proximal part of the catheter illustrated in FIG. 1.
FIG. 3 is an enlarged cross-sectional view of a distal part of the catheter illustrated in FIG. 1.
FIG. 4 is a cross-sectional view taken along line A-A in FIG. 1.
FIG. 5 is a cross-sectional view taken along line B-B in FIG. 1.
FIG. 6 is a cross-sectional view taken along line C-C in FIG. 1.
FIG. 7 is a view for describing an operation of deflecting a leading end of the catheter illustrated in FIG. 1.
FIG. 8 is a schematic view of a deflection mechanism when a deflecting lever in FIG. 7 is placed on the proximal side.
FIG. 9 is a schematic view of the deflection mechanism when the deflecting lever in FIG. 7 is placed on the distal side.
FIG. 10 is a schematic view of a catheter according to a second embodiment.
FIG. 11 is a schematic view illustrating a second tubular portion being housed in a first tubular portion of the catheter illustrated in FIG. 10.
FIG. 12 is a schematic view of a catheter according to a third embodiment.
FIG. 13 is a cross-sectional view taken along line D-D in FIG. 12.
FIG. 14 is a schematic view of a catheter according to a fourth embodiment.
FIG. 15 is a cross-sectional view taken along line E-E in FIG. 14.

### BEST MODE FOR CARRYING OUT THE INVENTION

Hereinafter, an embodiment of the invention will be described with reference to the drawings. In the drawings attached to this description, the scale and longitudinal and lateral dimensional ratios are exaggerated and changed from the actual ones for sake of simplicity.

### (First Embodiment)

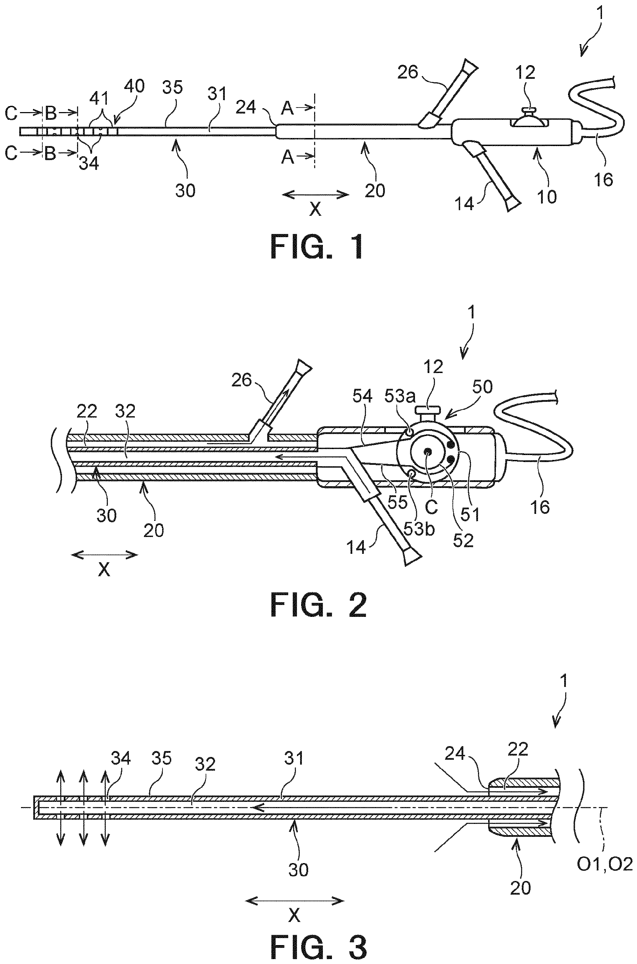

First, with reference to FIGS. 1 to 9, a catheter according to a first embodiment will be described.

As illustrated in FIG. 1, a catheter 1 according to this embodiment includes a handle 10, a first tubular portion 20 extending from the handle 10, a second tubular portion 30 passing through and extending from the first tubular portion 20, and a temperature sensor 40 that measures temperature.

First, the handle 10 will be described. In using the catheter 1, an operator holds the handle 10. The handle 10 may have a cylindrical shape. In the illustrated example, the handle 10 is provided with a deflecting lever 12, a fluid feeding port 14, and a cable 16.

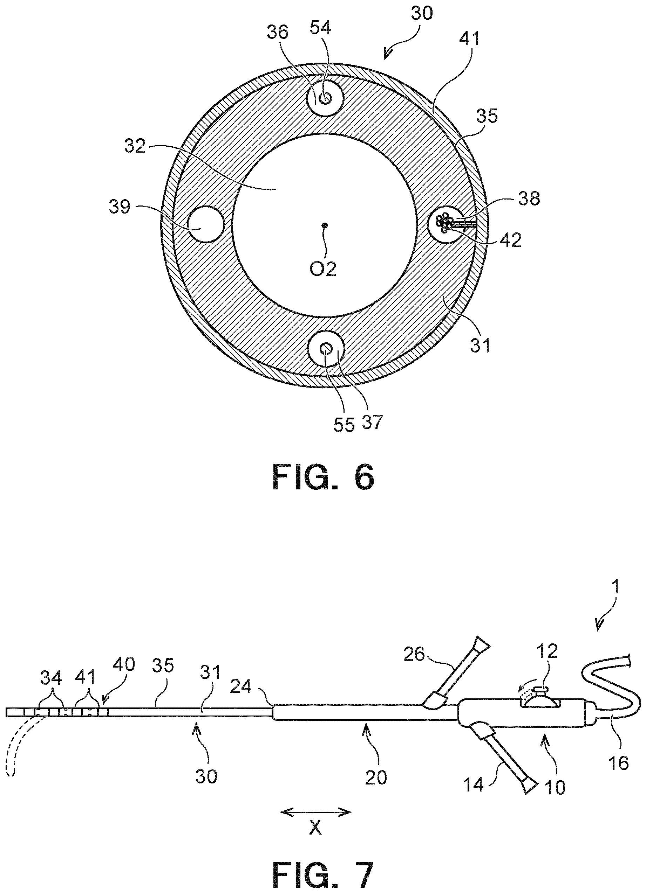

The deflecting lever 12 is operated by, for example, the operator with his/her finger and is inclined in a longitudinal direction of the catheter 1 (horizontal direction in FIG. 1, hereinafter referred to as "longitudinal direction X") when being pushed or pulled by the finger. As illustrated in FIG. 2, the deflecting lever 12 is connected to a deflection mechanism 50 to be described. As will be described later, for example, when the deflecting lever 12 is inclined toward the distal side in the longitudinal direction X (the left side in FIG. 7), the deflection mechanism 50 deflects a leading end of the catheter 1 (a leading end of an extension 31 of the second tubular portion 30 to be described later) toward one side (the lower side in FIG. 7) in a direction perpendicular to the longitudinal direction X, and when the deflecting lever 12 is inclined toward the proximal side in the longitudinal direction X (the right side in FIG. 7), the deflection mechanism 50 deflects the leading end of the catheter 1 toward the other side (the upper side in FIG. 7) in the direction perpendicular to the longitudinal direction X (see FIG. 7). Such a configuration reduces the risk of the catheter 1 straying into the air passage, particularly, when the catheter 1 is applied to the esophagus and inserted into the esophagus either transnasally or transorally. Furthermore, since the esophagus is not linear but meanders from the pharynx to the cardia of the stomach, the deflecting operation makes it possible to place a measuring unit 41 (to be described) at a site of interest in the esophagus.

Note that the term "proximal" represents the side of the catheter 1 closer to the operator in the longitudinal direction X. The term "distal" represents the side of the catheter 1 away from the operator in the longitudinal direction X.

The fluid feeding port 14 is used for injecting fluid through the catheter 1 and communicates with a second fluid channel 32 inside the second tubular portion 30 to be described. The fluid feeding port 14 may be connected through a tube to a feeding device (not illustrated) provided with a feeding pump, and the fluid may be injected through the catheter 1 by driving the feeding pump. In this case, the drive of the feeding pump is preferably controlled based on signals detected by the temperature sensor 40. Alternatively, the fluid feeding port 14 may be connected to a syringe (not illustrated) through a tube, and the fluid may be injected through the catheter 1 by the operator operating the syringe.

The cable 16 is used for connecting the catheter 1 and a gauge (not illustrated), and information detected by the temperature sensor 40 (for example, thermoelectromotive force) is input to the gauge through the cable 16 and converted into temperature information (in vivo temperature). With a display device, the converted temperature information is displayed outside, for example, on a main body of the gauge or an external device (such as monitor and display) connected to the gauge.

Next, the first tubular portion 20 and the second tubular portion 30 will be described. The first tubular portion 20 and the second tubular portion 30 are to be inserted into the esophagus or other internal organs of a patient when the catheter 1 is used. As illustrated in FIGS. 1 to 4, the second tubular portion 30 has an outside diameter smaller than an inside diameter of the first tubular portion 20. Furthermore, the second tubular portion 30 has a larger length than the first tubular portion 20 in the longitudinal direction X. The second tubular portion 30 passes through the first tubular portion 20. For this reason, the second tubular portion 30 has an extension 31 extending from a leading end of the first tubular portion 20. In the illustrated example, the first tubular portion 20 and the second tubular portion 30 are placed in such a manner that the central axis O1 of the first tubular portion 20 coincides with the central axis O2 of the second tubular portion 30.

As illustrated in FIGS. 2 to 6, the interior of the second tubular portion 30 is provided with the second fluid channel 32 through which the fluid flows. As illustrated in FIG. 2, a proximal end of the second tubular portion 30 (right end in FIG. 2) is connected to a distal end of the handle 10 (left end in FIG. 2). The interior of the second tubular portion 30 communicates with the interior of the handle 10, and the fluid injected from the fluid feeding port 14 flows into the second fluid channel 32 inside the second tubular portion 30.

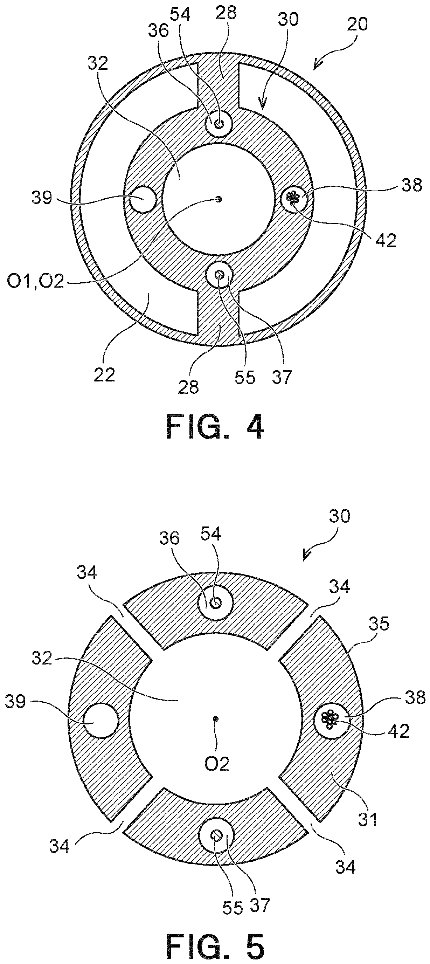

As illustrated in FIGS. 1 and 3, the extension 31 of the second tubular portion 30 is provided with a second opening 34. The second opening 34 leads to the interior of the second tubular portion 30. In other words, the second fluid channel 32 inside the second tubular portion 30 communicates with the outside of the second tubular portion 30 through the second opening 34. In the illustrated example, a plurality of second openings 34 is disposed on a side surface 35 of the extension 31 of the second tubular portion 30, being separated from each other in the longitudinal direction X (along the central axis O2 of the second tubular portion 30). Furthermore, as illustrated in FIG. 5, the plurality of second openings 34 is disposed on the side surface 35 of the extension 31 of the second tubular portion 30, being separated from each other in a circumferential direction around the central axis O2 of the second tubular portion 30. However, the invention is not limited to this embodiment, and one second opening 34 may be disposed on the side surface 35 of the extension 31 of the second tubular portion 30, or the second opening 34 may be disposed on a leading end of the second tubular portion 30. In a case where there is the plurality of second openings 34, the second openings 34 may have a substantially equal area. The fluid injected from the fluid feeding port 14 and flowing through the second fluid channel 32 inside the second tubular portion 30 is discharged from the second opening 34 to the outside of the second tubular portion 30 (the outside of the catheter 1). In other words, the second opening 34 feeds the fluid to the esophagus or other internal organs outside the second tubular portion 30. Accordingly, the fluid cools or warms the esophagus or other internal organs.

As illustrated in FIGS. 4 to 6, when the second tubular portion 30 is viewed in cross section perpendicular to an axial direction X, four pathways (lumens) 36, 37, 38, and 39 may be formed around the second fluid channel 32, that is, in a wall defining the second fluid channel 32. In other words, the four pathways may be formed in the second tubular portion 30. Among these pathways, the pathways 36 and 37 respectively allow pull wires 54 and 55 (to be described) to pass therethrough. Furthermore, the pathways 38 and 39 are thermocouple pathways that allow a thermocouple 42 of the temperature sensor 40 (to be described) to pass therethrough.

As illustrated in FIGS. 2 to 4, the interior of the first tubular portion 20 is provided with a first fluid channel 22 through which the fluid flows. More specifically, the first fluid channel 22 is formed inside the first tubular portion 20, that is, a space between the first tubular portion 20 and the second tubular portion 30. As illustrated in FIG. 2, a proximal end of the first tubular portion 20 (right end in FIG. 2) is connected to the distal end of the handle 10 (left end in FIG. 2). The interior of the first tubular portion 20 does not communicate with the interior of the handle 10 and the interior of the second tubular portion 30, and the fluid injected from the fluid feeding port 14 does not flow into the first fluid channel 22 inside the first tubular portion 20.

As illustrated in FIGS. 1 and 3, the first tubular portion 20 is provided with a first opening 24. The first opening 24 leads to the interior of the first tubular portion 20. In other words, the first fluid channel 22 inside the first tubular portion 20 communicates with the outside of the first tubular portion 20 through the first opening 24. In the illustrated example, the first opening 24 is disposed on the leading end of the first tubular portion 20, opening at the leading end of the first tubular portion 20. However, the invention is not limited to this embodiment, and the first opening 24 may be disposed on a side surface of the first tubular portion 20. Furthermore, more than one first opening 24 may be disposed on the side surface of the first tubular portion 20. The fluid outside the first tubular portion 20 (outside the catheter 1) is collected inside the first tubular portion 20 from the first opening 24. In other words, the fluid fed from the second opening 34 to an internal organ such as the esophagus is designed to flow into the first fluid channel 22 inside the first tubular portion 20 from the first opening 24 after cooling or heating the internal organ.

As illustrated in FIGS. 1 and 2, the side surface of the first tubular portion 20 on the proximal side is provided with a fluid collector 26. The fluid collector 26 communicates with the first fluid channel 22, and the fluid flowing through the first fluid channel 22 is collected by this fluid collector 26. The fluid collector 26 may be connected through a tube to an aspiration device (not illustrated) provided with an aspiration pump, and the fluid may be aspirated from the first opening 24 when the aspiration pump is driven to negatively reduce the pressure inside the first fluid channel 22. Alternatively, the fluid collector 26 may be connected to a syringe (not illustrated ) through a tube, and the fluid may be collected from the first opening 24 by an operator operating the syringe.

As illustrated in FIG. 4, an inner side surface of the first tubular portion 20 and an outer side surface of the second tubular portion 30 may be partially coupled via a connection 28, and the first tubular portion 20 and the second tubular portion 30 may be integrated. The first tubular portion 20 and the second tubular portion 30 may be formed seamlessly in an integrated manner, using the same material at the time of manufacturing. In particular, the first tubular portion 20 and the second tubular portion 30 may be integrally molded of the same material. In addition, the leading end of the first tubular portion 20 may be tapered.

In the longitudinal direction X, the first tubular portion 20 may have a length of 200 mm or more and 1000 mm or less. The first tubular portion 20 may have an inside diameter of 1.0 mm or more and 5.0 mm or less. The first tubular portion 20 may have an outside diameter of 1.7 mm or more and 6.0 mm or less. Furthermore, in the longitudinal direction X, the second tubular portion 30 may have a length of 300 mm or more and 1100 mm or less. The second tubular portion 30 may have an outside diameter of 2.3 mm or more and 4.0 mm or less. The first tubular portion 20 and the second tubular portion 30 may employ any material as long as the material is flexible that enables transnasal or transoral insertion into the body. Examples of such a flexible material include polyvinyl chloride, silicone, polyether block amide, polyurethane, nylon, polyolefin, polyamide and polyether polyamide. In addition, in order to check a site where the first tubular portion 20 and the second tubular portion 30 indwell in the body, it is more preferable that a material suitable for contrast radiography is mixed in the flexible material.

Furthermore, the fluid to be injected through the catheter 1 may employ any fluid as long as the fluid is used for controlling temperature of an internal organ, such as saline, dextrose in water, purified water and tap water. The fluid temperature may be controlled. For example, in heat-based catheter ablation, fluid to be used may have a temperature of 0°C or more and 10°C or less, and in cold-based catheter ablation, fluid to be used may have a temperature of 25°C or more and 37°C or less.

Next, the temperature sensor 40 will be described. The temperature sensor 40 measures temperature of an internal organ of a patient when the catheter 1 is used. The temperature sensor 40 includes the measuring unit 41 placed in the extension 31 of the second tubular portion 30 and the thermocouple 42 configured to measure temperature of the measuring unit 41.

The measuring unit 41 have heat conductivity and have a tubular shape. As illustrated in FIGS. 1 and 6, the measuring unit 41 may be disposed on the side surface 35 of the extension 31 of the second tubular portion 30. In the illustrated example, the measuring unit 41 is placed between the first opening 24 and the second opening 34 in the longitudinal direction X. Furthermore, a plurality of measuring units 41 is disposed on the side surface 35 of the extension 31 of the second tubular portion 30, being separated from each other in the longitudinal direction X (along the central axis O2 of the second tubular portion 30). Furthermore, the measuring unit 41 and the second opening 34 are placed alternately along the longitudinal direction X (the central axis O2 of the second tubular portion 30). However, the invention is not limited to this embodiment, and one measuring unit 41 may be disposed on the side surface 35 of the extension 31 of the second tubular portion 30, or the measuring unit 41 may be disposed on the leading end of the second tubular portion 30. The measuring unit 41 may employ any material as long as the material has high heat conductivity. Examples of the material include metallic materials such as iron, aluminum, stainless steel, and platinum alloys.

The thermocouple 42 includes two metal wires of different types. As illustrated in FIGS. 4 to 6, the thermocouple 42 passes through the thermocouple pathway 38 in the second tubular portion 30. The thermocouple 42 may pass through the thermocouple pathway 39. A terminal of the thermocouple 42 is connected to the main body of the gauge (not illustrated) through the cable 16 as described above. On the other hand, as illustrated in FIG. 6, a leading end of the thermocouple 42 penetrates an inner wall of the second tubular portion 30 to be connected to the measuring unit 41 at a position where the measuring unit 41 is placed in the longitudinal direction X. More specifically, the leading end of the thermocouple 42 is fixed to the measuring unit 41, being in contact with the measuring unit 41. The leading end of the thermocouple 42 may be fixed to the measuring unit 41 by, for example, soldering. In a case where there is the plurality of measuring units 41, a plurality of thermocouples 42 passes through the thermocouple pathways 38 and 39, and leading ends of the thermocouples 42 penetrate the inner wall of the second tubular portion 30 to be connected to the measuring units 41 at position where each measuring unit 41 is placed. The combination of the metal wires included in the thermocouple 42 is, for example, a copper wire and a constantan wire.

In using the catheter 1, when the second tubular portion 30 is inserted into the internal organ of the patient, temperature (heat) of the internal organ is transmitted to the measuring unit 41 placed in the extension 31 of the second tubular portion 30. The temperature sensor 40 measures temperature of the internal organ by measuring temperature of the measuring unit 41 with the thermocouple 42.

Note that the measuring unit 41 is not limited to a tubular shape and may have any other shape. Instead of providing the measuring unit 41 separately, the leading end of the thermocouple 42 may serve as a measuring unit. In other words, the measuring unit may include the leading end of the thermocouple 42.

The catheter 1 according to this embodiment may also include the deflection mechanism 50 that deflects the leading end of the extension 31 of the second tubular portion 30. As illustrated in FIG. 2, the deflection mechanism 50 is connected to the deflecting lever 12 and is placed inside the handle 10. The deflection mechanism 50 includes a rotor 51, a first pull wire 54, and a second pull wire 55. The rotor 51 is connected to the deflecting lever 12 and is rotatably supported by a rotating shaft C. The deflecting lever 12 inclined in the longitudinal direction X causes the rotor 51 to rotate about the rotating shaft C. The rotor 51 has a side surface provided with an inner race 52, a first protrusion 53a and a second protrusion 53b. The inner race 52 is placed inside the rotor 51 and protrudes from a central portion of the side surface of the rotor 51. Each of the first protrusion 53a and the second protrusion 53b protrudes from the side surface of the rotor 51 on the distal side (the left side in FIG. 2). The first protrusion 53a is placed on one side (upper side in FIG. 2) in the direction perpendicular to the longitudinal direction X, and the second protrusion 53b is placed on the other side (lower side in FIG. 2) in the direction perpendicular to the longitudinal direction X. One end of the first pull wire 54 and one end of the second pull wire 55 are connected to the side surface of the rotor 51. One end of the first pull wire 54 and one end of the second pull wire 55 are connected to the side surface of the rotor 51 on the proximal side (right side in FIG. 2). The first pull wire 54 is connected to a part on one side (upper side in FIG. 2) in the direction perpendicular to the longitudinal direction X, and the second pull wire 55 is connected to a part on the other side (lower side in FIG. 2) in the direction perpendicular to the longitudinal direction X. The first pull wire 54 and the second pull wire 55 both pass between the first protrusion 53a and the second protrusion 53b. As illustrated in FIG. 4 to 6, the pull wires 54 and 55 pass through the pull wire pathways 36 and 37, respectively, and the other ends of the pull wires 54 and 55 are connected to the leading end of the extension 31 of the second tubular portion 30. The pull wires 54 and 55 have predetermined tension between the rotor 51 and the leading end of the extension 31 of the second tubular portion 30.

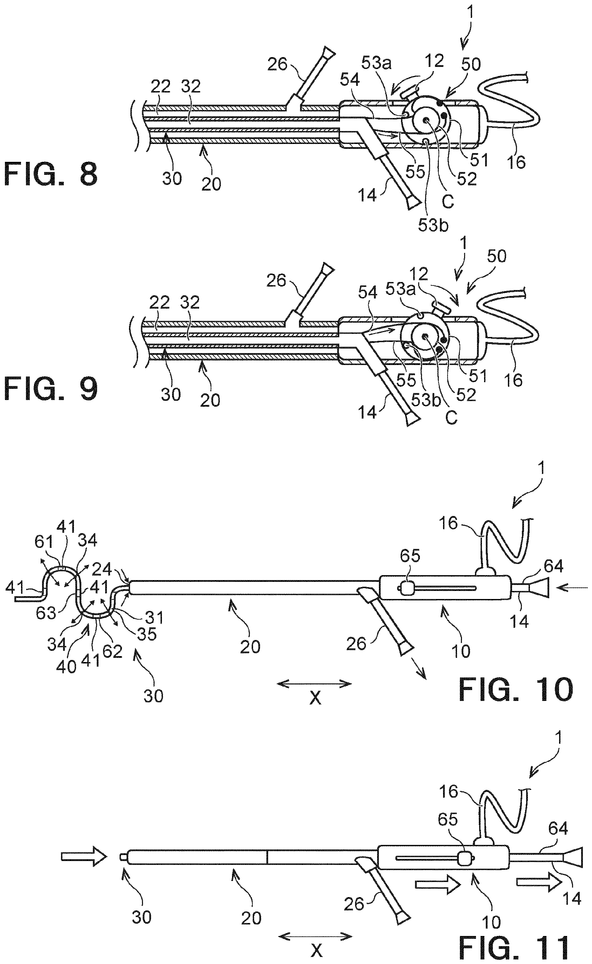

As illustrated in FIG. 7, the deflection mechanism 50 is configured to deflect the leading end of the extension 31 of the second tubular portion 30 toward one side (lower side in FIG. 7) in the direction perpendicular to the longitudinal direction X when the deflecting lever 12 is inclined toward the distal side (left side in FIG. 7). Furthermore, the deflection mechanism 50 is configured to deflect the leading end of the extension 31 of the second tubular portion 30 toward the other side (upper side in FIG. 7) in the direction perpendicular to the longitudinal direction X when the deflecting lever 12 is inclined toward the proximal side (right side in FIG. 7). More specifically, as illustrated in FIG. 2, when the deflecting lever 12 is upright, the tension of the first pull wire 54 and the tension of the second pull wire 55 are in equilibrium in the deflection mechanism 50. With this configuration, the leading end of the extension 31 of the second tubular portion 30 maintains the shape extending in the longitudinal direction X. As illustrated in FIG. 8, the deflecting lever 12 inclined toward the distal side causes the rotor 51 to rotate counterclockwise in FIG. 8 and loosens the first pull wire 54 to decrease the tension of the first pull wire 54 while pulling the second pull wire 55 in contact with an outer peripheral portion of the inner race 52 to increase the tension of the second pull wire 55. Consequently, the leading end of the extension 31 of the second tubular portion 30 is pulled by the second pull wire 55 and receives a force on one side (lower side in FIG. 7) in the direction perpendicular to the longitudinal direction X. Accordingly, the leading end of the extension 31 of the second tubular portion 30 is deflected toward one side (the leading end faces one side). Furthermore, as illustrated in FIG. 9, the deflecting lever 12 inclined toward the proximal side causes the rotor 51 to rotate clockwise in FIG. 9 and loosens the second pull wire 55 to decrease the tension of the second pull wire 55 while pulling the first pull wire 54 in contact with an outer peripheral portion of the inner race 52 to increase the tension of the first pull wire 54. Consequently, the leading end of the extension 31 of the second tubular portion 30 is pulled by the first pull wire 54 and receives a force on the other side (upper side in FIG. 7) in the direction perpendicular to the longitudinal direction X. Accordingly, the leading end of the extension 31 of the second tubular portion 30 is deflected toward the other side (the leading end faces the other side).

Hereinafter described are operations and effects of this embodiment having the aforementioned configuration.

The catheter 1 according to this embodiment is employed, for example, in catheter ablation using a separate ablation catheter. In the catheter ablation, a leading end of an ablation catheter inserted into the body ablates an internal organ to be treated such as a site of interest in the heart. At this time, temperature of other internal organs near the internal organ to be ablated also changes. The catheter 1 is used to cool an internal organ heated by the catheter ablation or to heat an internal organ cooled by the catheter ablation. Hereinafter described are operations and effects when temperature of the esophagus is controlled with the catheter 1 during the catheter ablation of a site of interest in the heart.

For example, during the catheter ablation, the catheter 1 is inserted into the esophagus from the nose or mouth of a patient lying on his/her back. More specifically, an operator inserts the first tubular portion 20 and the second tubular portion 30 of the catheter 1 into the esophagus of the patient while holding the handle 10 of the catheter 1. When the first tubular portion 20 and the second tubular portion 30 are inserted into the esophagus of the patient, the deflecting lever 12 may be operated to deflect the leading end of the extension 31 of the second tubular portion 30. Accordingly, the second tubular portion 30 is smoothly guided into the esophagus of the patient.

During the catheter ablation, temperature inside the esophagus of the patient is measured by the temperature sensor 40 of the catheter 1. More specifically, temperature (heat) of the esophagus is transmitted to the measuring unit 41 placed in the extension 31 of the second tubular portion 30, and the temperature transmitted to the measuring unit 41 is measured by the thermocouple 42 of the temperature sensor 40. The temperature measured by the temperature sensor 40 is displayed on the main body of the gauge (not illustrated), which enables the operator to monitor the temperature inside the esophagus of the patient.

When the measured temperature of the temperature sensor 40 is equal to or higher than a predetermined temperature (for example, equal to or higher than 39°C) or when the measured temperature of the temperature sensor 40 is equal to or lower than a predetermined temperature (for example, equal to or lower than 15°C), temperature-controlled fluid is injected through the catheter 1 from the fluid feeding port 14. The fluid injected from the fluid feeding port 14 flows into the second tubular portion 30 and flows through the second fluid channel 32 inside the second tubular portion 30. The fluid flowing through the second fluid channel 32 is discharged (ejected) from the second opening 34 placed in the extension 31 of the second tubular portion 30 to the outside of the second tubular portion 30, that is, to the esophagus. Accordingly, the esophagus of the patient is cooled or heated by the fluid.

The fluid discharged into the esophagus from the second opening 34 cools or heats the esophagus and is collected inside the first tubular portion 20 from the first opening 24, and then, flows through the first fluid channel 22 inside the first tubular portion 20. The fluid flowing through the first fluid channel 22 is collected by the fluid collector 26. In this manner, the fluid discharged from the second opening 34 is collected from the first opening 24.

According to this embodiment, the catheter 1 includes the temperature sensor 40, and the fluid discharged from the second opening 34 is collected from the first opening 24. With this configuration, when temperature of an internal organ becomes high or low, the fluid is discharged and collected by the catheter 1, which enables efficient temperature control of the internal organ. For this reason, there is no need to stop the catheter ablation temporarily to provide a cooling time. Consequently, treatment time of the catheter ablation is reduced, and patient burden is reduced.

Furthermore, according to this embodiment, the single catheter 1 has functions of temperature measurement, fluid discharge and fluid collection. Such a configuration avoids complication of the device and simplifies operations such as temperature measurement, fluid discharge and fluid collection.

Still further, according to this embodiment, the catheter 1 includes the first tubular portion 20 provided with the first opening 24 for fluid collection and the second tubular portion 30 provided with the second opening 34 for fluid discharge. With this configuration, it is easy to independently provide a channel through which the injected fluid flows (second fluid channel 32) and a channel through which the collected fluid flows (first fluid channel 22). Accordingly, it is possible to continuously inject and collect the fluid, which enables efficient temperature control of the internal organ.

Still further, according to this embodiment, the first opening 24 for fluid collection is placed on the proximal side of the second opening 34 for fluid discharge. With this configuration, the fluid discharged from the second opening 34 is prevented from flowing into the trachea on the proximal side of the esophagus when the catheter 1 is inserted into the esophagus of the patient. Accordingly, it is possible to reduce patient burden.

Still further, according to this embodiment, the measuring unit 41 is placed between the first opening 24 and the second opening 34 in the longitudinal direction X. Since the fluid is discharged from the second opening 34 and collected from the first opening 24, a portion between the first opening 24 and the second opening 34 is a main site of interest whose temperature is to be controlled. For this reason, placing the measuring unit 41 between the first opening 24 and the second opening 34 makes it possible to measure temperature of the site of interest with high accuracy. Consequently, temperature of the site of interest is perceived accurately, which enables temperature control of the internal organ more efficiently.

Still further, according to this embodiment, the first opening 24 opens at the leading end of the first tubular portion 20. With this configuration, for example, when the fluid is aspirated from the first opening 24 by an aspiration device, the first opening 24 is prevented from aspirating the inner wall surface of the esophagus or the like. For this reason, it is possible to suppress a decrease in aspiration force from the first opening 24, which enables efficient collection of the fluid. In addition, the first opening 24 for fluid collection opens towards the second opening 34 for fluid feeding, and even for this reason, the fluid fed from the second opening 34 into the body is efficiently collected.

Still further, according to this embodiment, the second opening 34 includes a plurality of openings disposed on the side surface 35 of the extension 31 of the second tubular portion 30, the openings being separated from each other along the central axis O2 of the second tubular portion 30. With this configuration, the fluid is ejected from the plurality of second openings 34 disposed on the side surface 35 of the extension 31 of the second tubular portion 30 to the inner wall surface of the esophagus or the like having high or low temperature. Accordingly, it is possible to quickly cool or heat the inner wall surface of the esophagus or the like, which enables temperature control of the internal organ more efficiently.

Still further, according to this embodiment, the temperature sensor 40 has the plurality of measuring units 41 placed alternately with the second opening 34 along the central axis O2 of the second tubular portion 30. With this configuration, a temperature distribution of the internal organ is perceived in more detail by the temperature sensor 40, and a part surrounding the temperature-measured site is cooled or heated by the fluid discharged from the second opening 34. Accordingly, temperature of the internal organ is controlled more efficiently.

Still further, according to this embodiment, the second opening 34 includes the plurality of openings disposed on the side surface 35 of the extension 31 of the second tubular portion 30, the openings being separated from each other in a circumferential direction around the central axis O2 of the second tubular portion 30. With this configuration, the fluid is sprayed from the plurality of second openings 34 to the inner wall surface of the esophagus or the like in a wide range in the circumferential direction. Accordingly, it is possible to uniformly cool or heat the inner wall surface of the esophagus or the like, which enables temperature control of the internal organ more efficiently.

Still further, according to this embodiment, the first tubular portion 20 and the second tubular portion 30 are integrated. That is, the first tubular portion 20 and the second tubular portion 30 are formed seamlessly, and typically, the first tubular portion 20 and the second tubular portion 30 are molded in an integrated manner. With this configuration, the second fluid channel 32 in the second tubular portion 30 is successfully prevented from being blocked by the first tubular portion 20. It is also possible to successfully prevent the first opening 24 from being closed by the second tubular portion 30. Accordingly, internal organ temperature is controlled stably.

Still further, according to this embodiment, the leading end of the first tubular portion 20 is tapered. With this configuration, for example, the leading end of the first tubular portion 20 is prevented from being caught in the nasal cavity or the pharynx when the first tubular portion 20 is inserted into the esophagus. Accordingly, the catheter 1 is smoothly guided into the esophagus.

The embodiment shows an example in which there is the plurality of second openings 34, the second openings 34 have a substantially equal area. However, the invention is not limited to this embodiment, and the second openings 34 may have different areas. Particularly, an area of a second opening 34 on the distal side may be larger than an area of another second opening 34 on the proximal side. In other words, one second opening 34 among the plurality of second openings 34 may have a larger area than that of another second opening 34 on the proximal side of the foregoing second opening 34. Alternatively, the second openings 34 may have an area increasing from the proximal side toward the distal side. In other words, any one second opening 34 among the plurality of second openings 34 may have an area equal to or larger than another second opening 34 on the proximal side of the foregoing second opening 34.

In a case where the second tubular portion 30 is provided with the plurality of second openings 34, the fluid tends to be discharged from the second opening 34 on the proximal side (on the upper side) more easily than from the second opening 34 on the distal side (on the lower side). For this reason, making an area of the second opening 34 on the distal side larger than an area of the second opening 34 on the proximal side uniforms an amount of fluid discharged from each second opening 34. Accordingly, the inner wall surface of the esophagus or the like is uniformly cooled or heated in the longitudinal direction, which enables temperature control of the internal organ more efficiently.

In addition, an area of one second opening 34 among the plurality of second openings 34 may be larger than an area of another second opening 34 on the proximal side of the foregoing second opening 34 and may be larger than an area of another second opening 34 on the distal side of the foregoing second opening 34. This configuration improves the temperature control function at a middle part of the extension 31 along the longitudinal direction X, thereby enabling temperature control of the internal organ more efficiently.

Still further, the embodiment shows an example in which the fluid discharged from the second opening 34 is collected from the first opening 24. However, the invention is not limited to this embodiment, and the fluid discharged from the first opening 24 may be collected from the second opening 34. In this case, the fluid flows against the direction of the embodiment.

Even in such a case, when temperature of the internal organ becomes high or low, the fluid is discharged and collected by the catheter 1, and temperature of the internal organ is efficiently controlled.

Furthermore, the embodiment shows an example in which the catheter 1 is inserted into the esophagus of a patient. However, the invention is not limited to this embodiment, and the catheter 1 may be inserted into any internal organs in need of temperature control.

An embodiment has been described with reference to specific examples, but the specific examples are not intended to limit the embodiment. The embodiment is implemented in various other forms, and various omissions, substitutions and changes in the forms may be made without departing from the gist of the invention.

### (Second Embodiment)

Next, with reference to FIGS. 10 and 11, a catheter according to a second embodiment will be described.

The second embodiment illustrated in FIGS. 10 and 11 is substantially equal to the first embodiment illustrated in FIGS. 1 to 9 except that an extension of a second tubular portion has a corrugated shape curved toward one side and another side. In FIGS. 10 and 11, the same parts as those in the first embodiment illustrated in FIGS. 1 to 9 are denoted by the same reference numerals and will not hereinafter be described in detail.

In this embodiment, as illustrated in FIG. 10, an extension 31 of a second tubular portion 30 has a corrugated shape curved toward one side (upper side in FIG. 10) and the other side (lower side in FIG. 10). More specifically, the corrugated shape has a first curved portion 61 curved toward one side, a second curved portion 62 curved toward the other side and an intermediate portion 63 between the first curved portion 61 and the second curved portion 62. In the illustrated example, the corrugated shape has one first curved portion 61 and one second curved portion 62, but the invention is not limited to this embodiment, and the corrugated shape may have two or more first curved portions 61 and/or two or more second curved portions 62. For example, the extension 31 of the second tubular portion 30 may be bent to be alternately folded back on one side and the other side in a direction perpendicular to longitudinal direction X. The second tubular portion 30 may include a flexible material that memorizes the corrugated shape. Examples of the material of a first tubular portion 20 and the second tubular portion 30 include thermoplastic materials such as polyvinyl chloride, silicone, polyether block amide, polyurethane, nylon, polyolefin, polyamide and polyether polyamide, and shape-memory alloys such as nickel titanium alloy.

In the illustrated example, a plurality of measuring units 41 is disposed on a side surface 35 of the extension 31 of the second tubular portion 30, being separated from each other along the central axis O2 of the second tubular portion 30. Furthermore, the measuring units 41 are placed alternately with second openings 34 along the central axis O2 of the second tubular portion 30. In the illustrated example, the measuring units 41 are placed in the first curved portion 61, the second curved portion 62 and the intermediate portion 63, respectively. The second openings 34 are placed between each measuring units 41, respectively. In other words, the second openings 34 are placed between the measuring unit 41 in the first curved portion 61 and the measuring unit 41 in the intermediate portion 63, and between the measuring unit 41 in the second curved portion 62 and the measuring unit 41 in the intermediate portion 63, respectively. However, the invention is not limited to this embodiment, and the measuring units 41 and the second openings 34 may be at any positions in the extension 31 of the second tubular portion 30.

In this embodiment, as illustrated in FIGS. 10 and 11, the second tubular portion 30 is movable relative to the first tubular portion 20. In the illustrated example, the first tubular portion 20 has a proximal end (right end in FIG. 10) connected to a distal end of the handle 10 (left end in FIG. 10). In contrast, the second tubular portion 30 has a proximal end not connected to the handle 10 but passing through the handle 10 and extending from a proximal end of the handle 10. In other words, the second tubular portion 30 has a proximal extension 64 extending from the proximal side of the handle 10. An operator pulls the proximal extension 64 toward the proximal side in the longitudinal direction X to move the second tubular portion 30 to the proximal side in the longitudinal direction X relative to the first tubular portion 20 as illustrated in FIG. 11. At this time, in a case where the second tubular portion 30 is made of a flexible material, the extension 31 of the second tubular portion 30 is deformed from the corrugated shape to a linear shape along the inner shape of the first tubular portion 20, thereby being housed in the second tubular portion 30. In this manner, the second tubular portion 30 is configured to be housed in the first tubular portion 20. From the state illustrated in FIG. 11, for example, when the proximal extension 64 is pushed and moved to the distal side in the longitudinal direction X, the second tubular portion 30 is moved to the distal side in the longitudinal direction X relative to the first tubular portion 20. Accordingly, as illustrated in FIG. 10, the extension 31 of the second tubular portion 30 is extended to the outside and forms the memorized corrugated shape.

As illustrated in FIGS. 10 and 11, the proximal extension 64 may be provided with a fluid feeding port 14 communicating with a second fluid channel 32 inside the second tubular portion 30. The operator injects fluid through the catheter 1 from the fluid feeding port 14.

In this embodiment, the handle 10 may be provided with a moving lever 65 instead of the deflecting lever 12 employed in the first embodiment. The moving lever 65 is movable in the longitudinal direction X by being operated by the operator with his/her finger or the like. The moving lever 65 is connected to the second tubular portion 30 inside the handle 10. With this configuration, when the moving lever 65 is moved in the longitudinal direction X, the second tubular portion 30 also moves in the longitudinal direction X together with the moving lever 65. In this manner, movement of the moving lever 65 in the longitudinal direction X also moves the second tubular portion 30 in the longitudinal direction X.

According to this embodiment, the extension 31 of the second tubular portion 30 has a corrugated shape curved toward one side and the other side. With this configuration, the fluid is fed from the second openings 34 to an internal organ such as the esophagus while the internal organ is expanded by the corrugated shape of the extension 31 of the second tubular portion 30. This makes it possible to cool or heat the internal organ more successfully, which enables temperature control of the internal organ more efficiently.

Furthermore, according to this embodiment, the second tubular portion 30 is movable relative to the first tubular portion 20 and configured to be housed in the first tubular portion 20. With this configuration, even though the extension 31 of the second tubular portion 30 has a corrugated shape, the second tubular portion 30 is smoothly guided to an internal organ of a patient such as the esophagus by being housed in the first tubular portion 20.

Still further, according to this embodiment, the measuring units 41 are placed in the first curved portion 61, the second curved portion 62 and the intermediate portion 63 of the corrugated shape, respectively. With this configuration, temperature of an internal organ such as the esophagus is measured with a planar spread, and a temperature distribution of the internal organ is measured with higher accuracy. Accordingly, it is possible to perceive temperature of the internal organ more accurately, which enables temperature control of the internal organ with higher efficiency.

### (Third Embodiment)

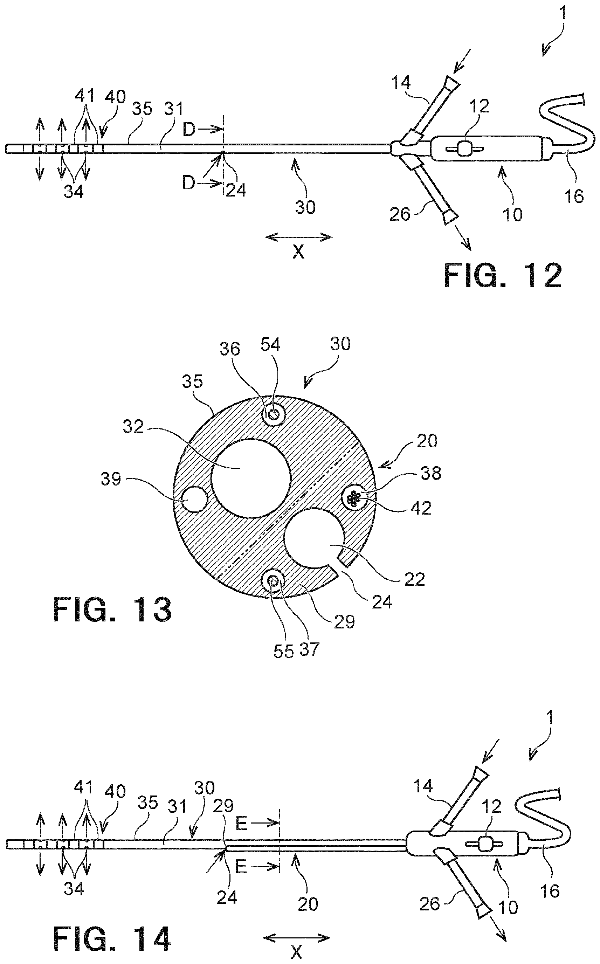

Next, with reference to FIGS. 12 and 13, a catheter according to a third embodiment will be described.

The third embodiment illustrated in FIGS. 12 and 13 is substantially equal to the first embodiment illustrated in FIGS. 1 to 9 except that a first tubular portion and a second tubular portion each have a substantially semicircular shape when viewed in a cross section perpendicular to an axial direction. In FIGS. 12 and 13, the same parts as those in the first embodiment illustrated in FIGS. 1 to 9 are denoted by the same reference numerals and will not hereinafter be described in detail.

In this embodiment, as illustrated in FIGS. 12 and 13, a first tubular portion 20 and a second tubular portion 30 each have a substantially semicircular shape when viewed in a cross section perpendicular to an axial direction X. The first tubular portion 20 and the second tubular portion 30 are integrated to form a circular shape. The first tubular portion 20 and the second tubular portion 30 may be formed seamlessly in an integrated manner, using the same material at the time of manufacturing. In particular, the first tubular portion 20 and the second tubular portion 30 may be integrally molded of the same material. In the illustrated example, a leading end 29 of the first tubular portion 20 is formed to have a cross section illustrated in FIG. 13. As illustrated in FIG. 12, the second tubular portion 30 includes an extension 31 extending beyond the leading end 29 of the first tubular portion 20. The extension 31 of the second tubular portion 30 has a circular cross section.

Similarly to the first embodiment, as illustrated in FIG. 13, the interior of the first tubular portion 20 is provided with a first fluid channel 22. In addition, the first tubular portion 20 is provided with a first opening 24 communicating with the interior of the first tubular portion 20. In the illustrated example, the first opening 24 is disposed on a side surface of the first tubular portion 20. The first fluid channel 22 inside the first tubular portion 20 communicates with the outside of the first tubular portion 20 through this first opening 24. On the other hand, the first fluid channel 22 inside the first tubular portion 20 also communicates with a fluid collector 26.

Similarly to the first embodiment, as illustrated in FIG. 13, the interior of the second tubular portion 30 is provided with a second fluid channel 32. As illustrated in FIG. 12, the second tubular portion 30 is provided with a second opening 34 communicating with the interior of the second tubular portion 30. In the illustrated example, a plurality of second openings 34 is disposed on a side surface 35 of the extension 31 of the second tubular portion 30, being separated from each other in the longitudinal direction X (along the central axis O2 of the second tubular portion 30). The second opening 34 is placed alternately with a measuring unit 41 along the longitudinal direction X (the central axis O2 of the second tubular portion 30). Furthermore, a plurality of second openings 34 is disposed on the side surface 35 of the extension 31 of the second tubular portion 30, being separated from each other in a circumferential direction around the central axis O2 of the second tubular portion 30. The second fluid channel 32 inside the second tubular portion 30 communicates with the outside of the second tubular portion 30 through this second opening 34. On the other hand, the second fluid channel 32 inside the second tubular portion 30 also communicates with a fluid feeding port 14.

In this embodiment, the fluid injected from the fluid feeding port 14 flows through the second fluid channel 32 inside the second tubular portion 30 and is discharged (ejected) from the second opening 34 to the outside of the second tubular portion 30, that is, to the esophagus. Accordingly, the esophagus of the patient is cooled or heated by the fluid. The fluid after cooling or heating the esophagus is collected inside the second tubular portion 30 from the first opening 24 and flows through the first fluid channel 22 inside the second tubular portion 30, thereby being collected by the fluid collector 26.

In other words, a catheter 1 according to this embodiment includes the first tubular portion 20 provided with the first opening 24 communicating with the interior of the first tubular portion 20; the second tubular portion 30 having the extension 31 extending beyond the leading end 29 of the first tubular portion 20 and provided with the second opening 34 communicating with the interior of the second tubular portion 30; and the temperature sensor 40 configured to measure temperature of the measuring unit 41 placed in the extension 31 of the second tubular portion 30, and the catheter 1 is configured to collect the fluid discharged from the second opening 34 from the first opening 24. Note that the fluid discharged from the first opening 24 may be collected from the second opening 34.

Even in this embodiment, the catheter 1 includes a temperature sensor 40, and the fluid discharged from the second opening 34 is collected from the first opening 24. Alternatively, the fluid discharged from the first opening 24 is collected from the second opening 34. With this configuration, when temperature of an internal organ becomes high or low, the fluid is discharged and collected by the catheter 1, which enables efficient temperature control of the internal organ.

In addition, according to this embodiment, the first tubular portion 20 and the second tubular portion 30 are integrated to form a circular shape when viewed in a cross section perpendicular to the axial direction X. This makes it possible to further simplify the configuration of the device and operations such as temperature measurement, fluid discharge and fluid collection. In addition, since no step is formed in the catheter 1, it is possible to prevent the catheter 1 from being caught in the nasal cavity or the pharynx, for example, when the catheter 1 is inserted into the esophagus. Accordingly, the catheter 1 is smoothly guided into the esophagus.

### (Fourth Embodiment)

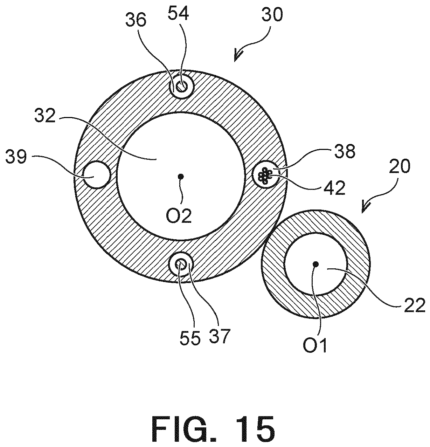

Next, with reference to FIGS. 14 and 15, a catheter according to a fourth embodiment will be described.

The fourth embodiment illustrated in FIGS. 14 and 15 is substantially equal to the first embodiment illustrated in FIGS. 1 to 9 except that a first tubular portion and a second tubular portion are extending in parallel. In FIGS. 14 and 15, the same parts as those in the first embodiment illustrated in FIGS. 1 to 9 are denoted by the same reference numerals and will not hereinafter be described in detail.

In this embodiment, as illustrated in FIGS. 14 and 15, a second tubular portion 30 does not pass through a first tubular portion 20, and the first tubular portion 20 and the second tubular portion 30 are extending in parallel. Similarly to the first embodiment, a proximal end (right end in FIG. 14) of the first tubular portion 20 is connected to a distal end (left end in FIG. 14) of a handle 10, and a proximal end of the second tubular portion 30 is connected to the distal end of the handle 10. Furthermore, the second tubular portion 30 is larger than the first tubular portion 20 in length in the longitudinal direction X. For this reason, the second tubular portion 30 has an extension 31 extending beyond a leading end 29 of the first tubular portion 20.

As illustrated in FIG. 15, a side surface of the first tubular portion 20 and a side surface of the second tubular portion 30 may be in contact with each other. However, the invention is not limited to this embodiment, and the side surface of the first tubular portion 20 and the side surface of the second tubular portion 30 may be separated from each other. In addition, the first tubular portion 20 and the second tubular portion 30 may be integrated. In this case, the first tubular portion 20 and the second tubular portion 30 may be formed in an integrated manner, using the same material at the time of manufacturing.

Similarly to the first embodiment, as illustrated in FIG. 15, the interior of the first tubular portion 20 is provided with a first fluid channel 22. As illustrated in FIG. 14, the first tubular portion 20 is provided with a first opening 24 communicating with the interior of the first tubular portion 20. In the illustrated example, the first opening 24 opens at the leading end 29 of the first tubular portion 20. The first fluid channel 22 inside the first tubular portion 20 communicates with the outside of the first tubular portion 20 through this first opening 24. On the other hand, the first fluid channel 22 inside the first tubular portion 20 also communicates with a fluid collector 26.

Similarly to the first embodiment, as illustrated in FIG. 15, the interior of the second tubular portion 30 is provided with a second fluid channel 32. As illustrated in FIG. 14, the second tubular portion 30 is provided with a second opening 34 communicating with the interior of the second tubular portion 30. In the illustrated example, a plurality of second openings 34 is disposed on a side surface 35 of the extension 31 of the second tubular portion 30, being separated from each other in the longitudinal direction X (along the central axis O2 of the second tubular portion 30). The second opening 34 is placed alternately with a measuring unit 41 along the longitudinal direction X (the central axis O2 of the second tubular portion 30). Furthermore, a plurality of second openings 34 is disposed on the side surface 35 of the extension 31 of the second tubular portion 30, being separated from each other in a circumferential direction around the central axis O2 of the second tubular portion 30. The second fluid channel 32 inside the second tubular portion 30 communicates with the outside of the second tubular portion 30 through this second opening 34. On the other hand, the second fluid channel 32 inside the second tubular portion 30 also communicates with a fluid feeding port 14.

In this embodiment, the fluid injected from the fluid feeding port 14 flows through the second fluid channel 32 inside the second tubular portion 30 and is discharged (ejected) from the second opening 34 to the outside of the second tubular portion 30, that is, to the esophagus. Accordingly, the esophagus of the patient is cooled or heated by the fluid. The fluid after cooling or heating the esophagus is collected inside the first tubular portion 20 from the first opening 24, and flows through the first fluid channel 22 inside the first tubular portion 20, thereby being collected by the fluid collector 26.

In other words, similarly to the third embodiment, a catheter 1 according to this embodiment includes the first tubular portion 20 provided with the first opening 24 communicating with the interior of the first tubular portion 20; the second tubular portion 30 having the extension 31 extending beyond the leading end 29 of the first tubular portion 20 and provided with the second opening 34 communicating with the interior of the second tubular portion 30; and the temperature sensor 40 configured to measure temperature of the measuring unit 41 placed in the extension 31 of the second tubular portion 30, and the catheter 1 is configured to collect fluid discharged from the second opening 34 from the first opening 24. Note that the fluid discharged from the first opening 24 may be collected from the second opening 34.

Even in this embodiment, the catheter 1 includes a temperature sensor 40, and the fluid discharged from the second opening 34 is collected from the first opening 24. Alternatively, the fluid discharged from the first opening 24 is collected from the second opening 34. With this configuration, when temperature of an internal organ becomes high or low, the fluid is discharged and collected by the catheter 1, which enables efficient temperature control of the internal organ.

In addition, according to this embodiment, the manufacturing of the catheter 1 is facilitated, and the manufacturing cost of the catheter 1 is reduced. Furthermore, it is possible to independently secure a channel through which the injected fluid flows and a channel through which the collected fluid flows. Accordingly, it is possible to freely control an injection pressure at the time of injecting the fluid and an aspiration pressure at the time of collecting the fluid, which enables efficient temperature control of the internal organ.

According to the aforementioned embodiments, it is possible to efficiently control temperature of an internal organ.

Although embodiments and modifications thereof have been described above, it is needless to say that two or more embodiments or modifications may be combined appropriately and that the embodiments and modifications may be employed at least partially.

## Claims

1. A catheter comprising:
a first tubular portion provided with a first opening communicating with an interior of the first tubular portion;
a second tubular portion passing through the first tubular portion, the second tubular portion having an extension extending from a leading end of the first tubular portion and provided with a second opening communicating with an interior of the second tubular portion; and
a temperature sensor configured to measure temperature of a measuring unit placed in the extension of the second tubular portion,
wherein the catheter is configured to collect fluid discharged from one of the first opening and the second opening from another one of the first opening and the second opening.

2. The catheter according to claim 1, wherein the measuring unit is placed between the first opening and the second opening in a longitudinal direction of the catheter.

3. The catheter according to claim 1 or 2, wherein the catheter is configured to collect the fluid discharged from the second opening from the first opening.

4. The catheter according to claim 3, wherein the second opening includes a plurality of openings disposed on a side surface of the extension of the second tubular portion, the openings being separated from each other along a central axis of the second tubular portion.

5. The catheter according to claim 4, wherein the temperature sensor has a plurality of the measuring units placed alternately with the second opening along the central axis of the second tubular portion.

6. The catheter according to claim 4 or 5, wherein one opening among the plurality of openings of the second opening has an area larger than an area of another opening disposed on the proximal side of the one opening.

7. The catheter according to any one of claims 3 to 6, wherein the second opening includes a plurality of openings disposed on a side surface of the extension of the second tubular portion, the openings being separated from each other in a circumferential direction around the central axis of the second tubular portion.

8. The catheter according to any one of claims 1 to 7, wherein the first tubular portion and the second tubular portion are integrated.

9. The catheter according to any one of claims 1 to 7, wherein the extension of the second tubular portion has a corrugated shape curved toward one side and another side.

10. The catheter according to claim 9, wherein the second tubular portion is movable relative to the first tubular portion and is configured to be housed in the first tubular portion.

11. The catheter according to claim 9 or 10,
wherein the corrugated shape has a first curved portion curved toward the one side, a second curved portion curved toward the other side and an intermediate portion between the first curved portion and the second curved portion, and
wherein the measuring unit is placed in the first curved portion, the second curved portion and the intermediate portion, respectively.

12. A catheter comprising:
a first tubular portion provided with a first opening communicating with an interior of the first tubular portion;
a second tubular portion having an extension extending beyond a leading end of the first tubular portion and provided with a second opening communicating with an interior of the second tubular portion; and
a temperature sensor configured to measure temperature of a measuring unit placed in the extension of the second tubular portion,
wherein the catheter is configured to collect fluid discharged from one of the first opening and the second opening from another one of the first opening and the second opening.
